# EUROPEAN PATENT SPECIFICATION

(11) **EP 1 383 532 B1**
(45) Date of publication and mention of the grant of the patent: **04.05.2011**
(21) Application number: 02719260.8
(22) Date of filing: 14.03.2002
(51) Int. Cl.: A61K 39/00, A61K 39/395

(54) **COMBINATION THERAPY**
KOMBINATIONSTHERAPIE
POLYTHERAPIE

(30) Priority: 02.04.2001 US 280805 P
(43) Date of publication of application: 28.01.2004
(60) Divisional application: 10176041.1
(73) Proprietor: Genentech, Inc., South San Francisco CA 94080-4990 (US)
(72) Inventor: GREWAL, Iqbal, Fremont, CA 94555 (US)
(74) Representative: Walton, Seán Malcolm
(86) International application number: PCT/US2002/008057
(87) International publication number: WO 2002/078766

(56) References cited:
- WO-A-01/34194
- WO-A-02/04021
- BENOIT NICOLE E ET AL: "Increased inhibition of proliferation of human B cell lymphomas following ligation of CD40, and either CD19, CD20, CD95 or surface immunoglobulin" IMMUNOPHARMACOLOGY, ELSEVIER SCIENCE PUBLISHERS BV, XX, vol. 35, no. 2, 1996, pages 129-139, XP002213373 ISSN: 0162-3109
- YE D. ET AL.: 'Augmentation of a humanized anti-HER2 mAb 4D5 induced growth inhibition by a human-mouse chimeric anti-EGF receptor mAb C225' ONCOGENE vol. 18, 1999, pages 731 - 738, XP001096335
- MURPHY W.J. ET AL.: 'Antibodies to CD40 prevent Epstein-Barr virus-mediated human B-cell lymphomagenesis in severe combined immune deficient mice given human peripheral blood lymphocytes' BLOOD vol. 86, no. 5, 01 September 1995, pages 1946 - 1953, XP002961507
- ILLIDGE T. ET AL.: 'Radioimmunotherapy in the pi-BCL1 B cell lymphoma model: efficacy depends on more than targeted irradiation alone' CANCER BIOTHER. RADIOPHARM. vol. 15, no. 6, December 2000, pages 581 - 591, XP002961506
- JAKOBSON E. ET AL.: 'Agonistic properties of anti-B cell antibodies purified on staphylococcal protein A may be due to contaminating protein A' J. IMMUNOL. METHODS vol. 152, no. 1, 31 July 1992, pages 49 - 57, XP002961505
- OTTAIANO A. ET AL.: 'CD40 activation as potential tool in malignant neoplasms' TUMORI vol. 88, no. 5, September 2002 - October 2002, pages 361 - 366, XP002961504
- ZIEBOLD J.L. ET AL.: 'Differential effects of CD40 stimulation on normal and neoplastic cell growth' ARCH. IMMUNOL. THER. EXP. (WARSZ) vol. 48, no. 4, 2000, pages 225 - 233, XP002961503
- ZHOU Z.H. ET AL.: 'An agonist anti-human CD40 monoclonal antibody that induces dendritic cell formation and maturation and inhibits proliferation of a myeloma cell line' HYBRIDOMA vol. 18, no. 6, December 1999, pages 471 - 478, XP002954467
- MALONEY D.G. ET AL.: 'IDEC-C2B8: results of a phase I multiple-does trial in patients with relapsed non-Hodgkin's lymphoma' J. CLIN. INVEST. vol. 15, no. 10, October 1997, pages 3266 - 3274, XP002936242

## Description

### Background of the Invention

### Field of the Invention

This invention relates to the treatment of diseases or disorders characterized by cells expressing the CD40 surface antigen and especially to the treatment of a neoplasm or autoimmune disease or disorder characterized by cells expressing the CD40 surface antigen. The invention provides compositions for use in methods for the treatment of various diseases or disorders characterized by cells expressing CD40 with a combination of an agent which arrests the growth of, destroys or causes the deletion of cells expressing CD40 and a second agent which arrests the growth of, destroys or causes the deletion of cells expressing the CD20 surface antigen. Pharmaceutical compositions and articles of manufacture such as kits comprising the agents and combinations thereof are also provided.

### Description of Related Disclosures

CD40 is a type I integral membrane glycoprotein and a member of the tumor necrosis factor (TNF) receptor superfamily. CD40 is expressed on a variety of cell types including normal and neoplastic B cells, interdigitating cells, basal epithelial cells and carcinomas. It is also present on macrophages, some endothelial cells and follicular dendritic cells. CD40 is expressed early in B cell ontogeny, appearing on B cell precursors subsequent to the appearance of CD10 and CD19 but prior to CD20 and surface immunoglobulin (Ig) (Uckun et al., (1990) Blood 15:2449). Although early reports indicated that CD40 was lost upon terminal differentiation of B cells into plasma cells, it has been detected on tonsil and bone marrow derived plasma cells (Pellat-Decounynck et al., (1994) Blood 84:2597).

The interaction of CD40 with its ligand, CD40L (also referred to as gp39) is central to the development of both humoral and cell mediated immune responses. CD40L is a transmembrane protein expressed predominantly on activated T lymphocytes. Like TNF proteins, the structure of the CD40L is a noncovalent trimer. CD40 mediated signaling appears to be a requirement for B cell proliferation, isotype switching, germinal center formation and memory B cell commitment in response to T cell dependent antigens. Receptor binding of CD40L results in CD40 multimerization, the generation of activation signals (for antigen presenting cells such as dendritic cells, monocytes and B cells) and the generation of growth and differentiation signals (for cytokine activated fibroblasts and epithelial cells). While the signaling pathways utilized by CD40 molecule in the regulation of cell fate have not been completely elucidated, CD40 signals are transduced from the multimerized receptor via recruitment of a series of TNF receptor associated factors ("TRAFs") (Kehry, (1996) J. Immunol. 156:2345-2348). Subsets of TRAFS interact differentially with TNF family members, including CD40 to provide stimuli to a wide variety of downstream pathways. TRAF1 and TRAF2 are implicated in the modulation of apoptosis (Speiser et al., (1997) J. Exp. Med. 185:1777-1783; Yeh et al., (1997) Immunity 7:715-725). TRAFs 2, 5 and 6 participate in proliferation and activation events. In normal B cells, binding of CD40 recruits TRAF2 and TRAF3 to the receptor complex and induces down regulation of other TRAFs (Kuhune et al., (1997) J. Exp. Med. 186:337-342).

Apoptosis and CD40 mediated signaling are closely linked during B cell development and differentiation. A primary function of apoptosis in B cells is the clonal deletion of immature B cells thought to result from extensive cross linking of surface Ig in immature B cells. The fate of mature B cells is also modulated by a combination of signaling through surface Ig and signals derived from activated T cells, presumably mediated by CD40L molecules. A combination of signals from surface Ig and CD40 have been shown to override the apoptotic pathway and maintain germinal center B cell survival. This rescue from apoptosis in germinal centers is critical for the development of high affinity antibody producing memory B cells.

In both T and B cell malignancies, antitumor effects (growth arrest with or without apoptosis) often result when malignant cells are exposed to stimuli that lead to activation of normal lymphocytes. This activation induced growth arrest has been observed with signals through either antigen receptors or costimulatory receptors (Ashwell et al., (1987) Science 237:61; Bridges et al., (1987) J. Immunol. 139:4242; Page and Defranco (1988) J. Immunol. 140:3717 and Beckwith et al., (1990) J. Natl. Cancer Inst. 82:501). CD40 simulation by either antibody or soluble ligand directly inhibits B cell lymphoma growth (Funakoshi et al., (1994) Blood 83:2787-2794).

Monoclonal antibodies (mAb) directed against CD40 have been described (Katira et al., "CD40 Workshop Panel Report" in Leukocyte Typing V, Schlossman et al., (Eds) 1995; 1:547-550). For example, two mAb, CD40.7 (M2) and CD40.8 (M3), were selected based upon their ability to inhibit the binding of CD40 to cells expressing CD40L (Fanslow et al., Leukocyte Typing V, Schlossman et al., (Eds) 1995, 1:555-556). CD40 stimulation by mAb M2 and M3 inhibits growth of several B-cell lymphomas and induces regression of established tumors in vivo (Funakoshi et al., (1994) Blood 83:2787-2794; Funakoshi et al., (1996) J. Immunol. 19:93-101). Single chain immunotoxins based upon mAb G28-5 selectively kill CD40 expressing malignant cells in vitro (Francisco et al., (1997) J Biol. Chem. 39:24165-24169). International Publication Number WO 00/75348 describes the use of recombinant forms of the CD40 directed antibody S2C6 in the treatment of various disorders including cancer. In addition to delivering a stimulatory signal, the antibody enhances the interaction between CD40 and CD40L. International Publication Number WO 95/17202 describes the use of antibodies that bind CD40 in the proper conformation in the treatment or prevention of disease characterized by neoplastic cells that express CD40.

The CD20 antigen (also called human B-lymphocyte-restricted differentiation antigen, Bp35) is a hydrophobic transmembrane protein with a molecular weight of approximately 35 kD located on pre-B and mature B lymphocytes (Valentine et al. J. Biol. Chem. 264(19):11282-11287 (1989); and Einfeld et al. EMBO J. 7(3):711-717 (1988)). The antigen is also expressed on greater than 90% of B cell non-Hodgkin's lymphomas (NHL) (Anderson et al. Blood 63(6):1424-1433 (1984)), but is not found on hematopoietic stem cells, pro-B cells, normal plasma cells or other normal tissues (Tedder et al. J. Immunol. 135(2):973-979 (1985)). CD20 regulates an early step(s) in the activation process for cell cycle initiation and differentiation (Tedder et al., supra) and possibly functions as a calcium ion channel (Tedder et al. J. Cell. Biochem. 14D:195 (1990)).

An anti-CD20 antibody, rituximab (RITUXAN® brand), is a genetically engineered chimeric murine/human monoclonal antibody directed against the CD20 antigen. Rituximab is the antibody called "C2B8" in US Patent No. 5,736,137 issued April 7, 1998 (Anderson et al.). The antibody is indicated for the treatment of patients with relapsed or refractory low-grade or follicular, CD20 positive, B cell non-Hodgkin's lymphoma. An open study of B-lymphocyte depletion through treatment with rituximab was conducted in rheumatoid arthritis patients the results suggesting that B-lymphocyte depletion maybe an effective therapy (Edwards and Cambridge (2001) Rheumatology 40:205-211). In vitro mechanism of action studies have demonstrated that the antibody binds human complement and lyses lymphoid B cell lines through complement-dependent cytotoxicity (CDC) (Reff et al. Blood 83(2) :435-445 (1994)). Additionally, it has significant activity in assays for antibody-dependent cellular cytotoxicity (ADCC). More recently, it has been shown to have anti-proliferative effects in tritiated thymidine incorporation assays and to induce apoptosis directly, while other CD20 antibodies do not (Maloney et al. Blood 88(10):637a (1996)). Synergy between Rituximab and chemotherapies and toxins has also been observed experimentally. In particular, Rituximab sensitizes drug-resistant human B cell lymphoma cell lines to the cytotoxic effects of doxorubicin, CDDP, VP-16, diphtheria toxin and ricin (Demidem et al. Cancer Chemotherapy & Radiopharmaceuticals 12(3):177-186 (1997)). In vivo, preclinical studies have shown that it depletes B cells from the peripheral blood, lymph nodes, and bone marrow of cynomolgus monkeys, presumably through complement and cell-mediated processes (Reff et al. Blood 83(2):435-445 (1994)).

### Summary of the Invention

The present invention encompasses compositions for use in the treatment of diseases and disorders characterized by cells expressing the CD40 surface antigen. In preferred aspects the invention relates to compositions for use in the treatment of diseases or disorders where B cell growth arrest or depletion provides a beneficial outcome such as slowing of disease progression, alleviation of one or more symptoms of a disease or remission, prevention or cure of the disease or disorder. Particular aspects relate to the treatment of various neoplastic diseases or disorders such as various hematological malignancies and certain other diseases or disorders where B cell depletion is indicated, such as for example, various autoimmune disorders such as rheumatoid arthritis and lupus erythrematosus. In particular embodiments, the invention provides compositions for use in methods for the treatment of various diseases or disorders characterized by cells expressing CD40 comprising the administration of an agent which arrests the growth of or causes the deletion of cells expressing CD40 in combination with a second agent which arrests the growth of or causes the deletion of cells expressing the CD20 membrane antigen.

According to a preferred embodiment, the treatment is of a neoplastic disease or disorder or an autoimmune disease or disorder characterized by cells expressing CD40. The method comprises the steps of administering to a mammal in need thereof a therapeutically effective amount of an agent which arrests the growth of or causes the deletion of cells expressing the CD40 membrane glycoprotein in combination with an agent which arrests the growth of or causes the deletion of cells expressing the CD20 membrane antigen. According to preferred aspects, the agent which arrests the growth of or causes the deletion of cells expressing the CD40 antigen is an agent which binds to the cell surface CD40 glycoprotein such as an antibody or other ligand directed to the receptor such as the CD40 ligand trimer. The agent which arrests the growth of or causes the deletion of cells expressing the CD20 membrane antigen is an antibody directed against CD20 which binds to CD20 and arrests the growth or causes the depletion of CD20 expressing cells.

Preferred aspects of the invention provide compositions for the treatment of a neoplastic disease or disorder characterized by cells which express the CD40 surface antigen, e.g. for the treatment of various hematological malignancies and various solid tumors. Preferred aspects of the invention provide for the treatment of a lymphoma or a myeloma characterized by cells expressing the CD40 surface antigen, e.g. a non-Hodgkin's type lymphoma and multiple myeloma.

Kits and articles of manufacture are also described. Kits and articles of manufacture preferably include:
(a) one or more containers;
(b) a label on each said container; and
(c) a first and second composition comprising an active agent contained within said containers; wherein the compositions are effective for treating a disease or disorder characterized by cells expressing CD40, such as a neoplastic or autoimmune disease or disorder, the label on said container may indicates that the composition can be used for treating a such a disorder, and the active agent in said first composition comprises an agent which arrests the growth of or causes the deletion of cells expressing the CD40 antigen and the active agent in said second composition comprises an agent which arrests the growth of or causes the deletion of cells expressing the CD20 antigen. The agents are antibodies. The kits optionally include accessory components such as an additional container comprising a pharmaceutically-acceptable buffer and instructions for using the compositions to treat a particular disease or disorder.

### Brief Description of the Drawings

Figure 1. Comparison of antitumor activity of humanized anti-CD40 antibody and an anti-CD20 antibody (RITUXAN® brand) against Ramos lymphoma transplanted in SCID mice.
Figure 2. Comparison of antitumor activity of a murine anti-CD40 antibody (SGN-14) and an anti-CD20 antibody (RITUXAN® brand) against Ramos lymphoma transplanted in SCID mice.
Figure 3. Antitumor activity of an anti-CD20 antibody (RITUXAN® brand) and humanized anti-CD40 antibody against IM9,(CD20+, CD40+ Multiple myeloma) transplanted in SCID mice.
Figure 4. Combination of an anti-CD20 antibody (RITUXAN® brand) and a murine anti-CD40 antibody (SGN-14) for antitumor activity against H.S. Sultan, (CD20+, CD40+Multiple myeloma) transplanted in SCID mice.
Figure 5. Anti-tumor activity of a murine anti-CD40 antibody (SGN-14) and an anti-CD20 antibody (RITUXAN® brand) on RITUXAN® brand resistant Ramos lymphoma transplanted in SCID mice.

### Detailed Description of the Preferred Embodiments

### Definitions

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity determining regions (CDRs) or hypervariable regions both in the light-chain and the heavy-chain variable domains. The more highly conserved portions of variable domains are called the framework region (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FRs and, with the CDRs from the other chain, contribute to the formation of the antigen-binding site of antibodies (see Kabat et al., NIH Publ. No.91-3242, Vol. I, pages 647-669 [1991]). The constant domains are not involved directly in binding an antibody to an antigen, but exhibit various effector functions, such as participation of the antibody in antibody dependent cellular cytotoxicity.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2. The heavy-chain constant domains that correspond to the different classes of immunoglobulins are called α, δ, ε, γ and µ, respectively. The subunit structures and three-dimensional configurations of different classes of immunoglobulins are well known.

The term "antibody" is used in the broadest sense and specifically covers intact monoclonal antibodies, polyclonal antibodies, multispecific antibodies (e.g. bispecific antibodies) formed from at least two intact antibodies, and antibody fragments so long as they exhibit the desired biological activity.

"Antibody fragments" comprise a portion of an intact antibody, preferably the antigen binding or variable region of the intact antibody. Examples of antibody fragments include Fab, Fab', F(ab')2, and Fv fragments; diabodies; linear antibodies (Zapata et al., Protein Eng. 8(10):1057-1062 [1995]); single-chain antibody molecules; and multispecific antibodies formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 [1984]).

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies are human immunoglobulins (recipient antibody) in which residues from a complementarity determining region (CDR) of the recipient are replaced by residues from a CDR of a non-human species (donor antibody) such as mouse, rat or rabbit having the desired specificity, affinity, and capacity. In some instances, framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDRs correspond to those of a non-human immunoglobulin and all or substantially all of the FRs are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 (1988); and Presta, Curr. Op. Struct. Biol., 2:593-596 (1992). The humanized antibody includes a PRIMATIZED™ antibody wherein the antigen-binding region of the antibody is derived from an antibody produced by immunizing macaque monkeys with the antigen of interest.

"Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains which enables the sFv to form the desired structure for antigen binding. For a review of sFv see Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia. More particular examples of such cancers include squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, breast cancer, colon cancer, colorectal cancer, endometrial carcinoma, salivary gland carcinoma, kidney cancer, prostate cancer, vulval cancer, thyroid cancer, hepatic carcinoma and various types of head and neck cancer.

The "CD20" antigen is a 35 kDa, non-glycosylated phosphoprotein found on the surface of greater than 90% of B cells from peripheral blood or lymphoid organs. CD20 is expressed during early pre-B cell development and remains until plasma cell differentiation. CD20 is present on both normal B cells as well as malignant B cells. Other names for CD20 in the literature include "B-lymphocyte-restricted antigen" and "Bp35". The CD20 antigen is described in Clark et al. PNAS (USA) 82:1766 (1985), for example.

"CD40" is meant to refer to the 50kD glycoprotein expressed on the surface of normal and neoplastic B cells acting as a receptor for signals involved in cellular proliferation and differentiation and sometimes referred to as Bp50 (Ledbetter et al., (1987) H, Immunol., 138:788-795). A cDNA encoding CD40 has been isolated from a library prepared from Burkitt lymphoma cell line Raji (Stamenkovic et al., (1989) EMBO J. 8:1403). A cell that expresses CD40 is any cell that is characterized by the surface expression of CD40, including but not limited to normal and neoplastic B cells, interdigitating cells, basal epithelial cells and carcinoma cells, macrophages, endothelial cells, follicular dendritic cells, tonsil cells and bone marrow derived plasma cells.

The term "cytotoxic agent" as used herein refers to a substance that arrests the growth of, inhibits or prevents the function of cells and/or causes deletion of cells. The term is intended to include radioactive isotopes (e.g. I131, I125, Y90 and Re186), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin gamma1I and calicheamicin phiI1, see, e.g., Agnew, Chem Intl. Ed. Engl., 33:183-186 (1994); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (Adriamycin™) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK®; razoxane; rhizoxin; sizofiran; spirogermanium; tenuazonic acid; triaziquone; 2, 2',2"-trichlorotriethylamine: trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, NJ) and doxetaxel (TAXOTERE®, Rh6ne-Poulenc Rorer, Antony, France); chlorambucil; gemcitabine (Gemzar™); 6-thioguanine; mercaptopurine; methotrexate; platinum analogs such as cisplatin and carboplatin; vinblastine; platinum; etoposide (VP-16); ifosfamide; mitoxantrone; vincristine; vinorelbine (Navelbine™); novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included in this definition are anti-hormonal agents that act to regulate or inhibit hormone action on tumors such as anti-estrogens and selective estrogen receptor modulators (SERMs), including, for example, tamoxifen (including NolvadexTM), raloxifene, droloxifene, 4-hydroxytamoxifen, trioxifene, keoxifene, LY117018, onapristone, and toremifene (Fareston™); aromatase inhibitors that inhibit the enzyme aromatase, which regulates estrogen production in the adrenal glands, such as, for example, 4(5)-imidazoles, aminoglutethimide, megestrol acetate (Megace™), exemestane, formestane, fadrozole, vorozole (Rivisor™), letrozole (Femara™), and anastrozole (Arimidex™); and anti-androgens such as flutamide, nilutamide, bicalutamide, leuprolide, and goserelin; and pharmaceutically acceptable salts, acids or derivatives of any of the above.

A "disorder" is any condition that would benefit from treatment with the combination therapy described herein. This includes chronic and acute disorders or diseases including those pathological conditions which predispose the mammal to the disorder in question. Non-limiting examples of disorders to be treated herein include cancer, heamotological malignancies, benign and malignant tumors; leukemias and lymphoid malignancies and inflammatory, angiogenic and immunologic disorders.

An agent which "arrests the growth of" or a "growth inhibitory agent" when used herein refers to a compound or composition which inhibits growth or proliferation of a cell, especially a neoplastic cell type expressing the CD40 antigen or the CD20 antigen as required. Thus, the growth inhibitory agent is one which for example, significantly reduces the percentage of neoplastic cells in S phase.

The term "intravenous infusion" refers to introduction of an agent into the vein of an animal or human patient over a period of time greater than approximately 15 minutes, generally between approximately 30 to 90 minutes.

The term "intravenous bolus" or "intravenous push" refers to drug administration into a vein of an animal or human such that the body receives the drug in approximately 15 minutes or less, generally 5 minutes or less.

The term "subcutaneous administration" refers to introduction of an agent under the skin of an animal or human patient, preferable within a pocket between the skin and underlying tissue, by relatively slow, sustained delivery from a drug receptacle. The pocket may be created by pinching or drawing the skin up and away from underlying tissue.

"Mammal" for purposes of treatment refers to any animal classified as a mammal, including humans, domestic and farm animals, and zoo, sports, or pet animals, such as dogs, horses, cats and cows to name but a few.

The term "package insert" is used to refer to instructions customarily included in commercial packages of therapeutic products, that contain information about the indications, usage, dosage, administration, contraindications and/or warnings concerning the use of such therapeutic products.

The humanized anti-CD20 antibody referred to as the "RITUXAN® brand" anti-CD20 antibody is a genetically engineered chimeric murine/human monoclonal antibody directed against the CD20 antigen. Rituximab is the antibody called "C2B8" in US Patent No. 5,736,137 issued April 7, 1998. The RITUXAN® brand of C2B8 antibody is indicated for the treatment of patients with relapsed or refractory low-grade or follicular, CD20 positive, B cell non-Hodgkin's lymphoma.

The term "subcutaneous infusion" refers to introduction of a drug under the skin of an animal or human patient, preferably within a pocket between the skin and underlying tissue, by relatively slow, sustained delivery from a drug receptacle for a period of time including, but not limited to, 30 minutes or less, or 90 minutes or less. Optionally, the infusion may be made by subcutaneous implantation of a drug delivery pump implanted under the skin of the animal or human patient, wherein the pump delivers a predetermined amount of drug for a predetermined period of time, such as 30 minutes, 90 minutes, or a time period spanning the length of the treatment regimen.

The term "subcutaneous bolus" refers to drug administration beneath the skin of an animal or human patient, where bolus drug delivery is preferably less than approximately 15 minutes, more preferably less than 5 minutes, and most preferably less than 60 seconds. Administration is preferably within a pocket between the skin and underlying tissue, where the pocket

The term "therapeutically effective amount" is used to refer to an amount of an active agent having a growth arrest effect or causes the deletion of the cell. Preferably, the therapeutically effective amount has apoptotic activity, or is capable of inducing cell death. In particular aspects, the therapeutically effective amount refers to a target serum concentration that has been shown to be effective in, for example, slowing disease progression. Efficacy can be measured in conventional ways, depending on the condition to be treated. For example, in neoplastic diseases or disorders characterized by cells expressing CD40, efficacy can be measured by assessing the time to disease progression (TTP), or determining the response rates (RR).

The terms "treatment" and "therapy" and the like as used within the context of the present invention, are meant to include therapeutic as well as prophylactic, or suppressive measures for a disease or disorder leading to any clinically desirable or beneficial effect, including but not limited to alleviation of one or more symptoms, regression, slowing or cessation of progression of the disease or disorder. Thus, for example, the term treatment includes the administration of an agent prior to or following the onset of a symptom of a disease or disorder thereby preventing or removing all signs of the disease or disorder. As another example, the term includes the administration of an agent after clinical manifestation of the disease to combat the symptoms of the disease. Further, administration of an agent after onset and after clinical symptoms have developed where administration affects clinical parameters of the disease or disorder, such as the degree of tissue injury or the amount or extent of metastasis, whether or not the treatment leads to amelioration of the disease, comprises "treatment" or "therapy" within the context of the invention.

### Modes for Carrying out the Invention

The present invention provides compositions for the treatment of a variety of diseases or disorders characterized by cells expressing the CD40 surface antigen by administration of an agent which arrests the growth or causes the deletion of cells expressing CD40 in combination with an agent which arrests the growth of or causes the deletion of cells expressing the CD20 antigen, as defined in claim 1 and claim 3.

According to the present invention, an agent which arrests the growth of or causes the deletion of cells expressing CD40 functions by any mechanism, including by binding the CD40 membrane antigen. Therefore, appropriate agents, also referred to herein as "CD40 specific agent" herein may arrest the growth of cells or the deletion of cells expressing CD40 by any mechanism of action and the invention in not meant to be limited by the mode of action of the agent. Therefore any agent which blocks proliferation or otherwise arrests the growth of a cell or causes its depletion, death or otherwise its deletion through binding the CD40 membrane antigen is an appropriate agent within the context of the invention.

By way of example, it is known that in both T and B cell malignancies, anti-tumor effects (growth arrest with or without deletion or apoptosis) often result when malignant cells are exposed to stimuli that lead to activation of normal lymphocytes. This activation induced growth arrest has been observed with signals through either antigen receptors or costimulatory receptors (Ashwell et al., (1987) Science 237:61; Bridges et al., (1987) J. Immunol. 139:4242; Page and Defranco (1988) J. Immunol. 140:3717 and Beckwith et al., (1990) J. Natl. Cancer Inst. 82:501). CD40 stimulation by either antibody or soluble ligand directly inhibits B cell lymphoma growth (Funakoshi et al., (1994) Blood 83:2787-2794). Agents directed against the CD40 membrane antigen and which inhibit malignant cell growth in such a way are an example of appropriate agents within the context of the invention.

Specific examples include but are not limited to monoclonal antibodies (mAb) directed against CD40. Such antibodies have been described (Katira et al., "CD40 Workshop Panel Report" in Leukocyte Typing V, Schlossman et al., (Eds) 1995; 1:547-550). For example, two mAb, CD40.7 (M2) and CD40.8 (M3), were selected based upon their ability to inhibit the binding of CD40 to cells expressing CD40L (Fanslow et al., Leukocyte Typing V, Schlossman et al., (Eds) 1995, 1:555-556). CD40 stimulation by mAb M2 and M3 inhibits growth of several B-cell lymphomas and induces regression of established tumors in vivo (Funakoshi et al., (1994) Blood 83:2787-2794; Funakoshi et al., (1996) J. Immunol. 19:93-101). Single chain immunotoxins based upon mAb G28-5 selectively kill CD40 expressing malignant cells in vitro (Francisco et al., (1997) J Biol. Chem. 39:24165-24169). International Publication Number WO 00/75348 describes the use of recombinant forms of the CD40 directed antibody S2C6 in the treatment of various disorders including cancer. In addition to delivering a stimulatory signal, the antibody enhances the interaction between CD40 and CD40L. International Publication Number WO 95/17202 describes the use of ligands that bind CD40 in the proper conformation in the treatment or prevention of disease characterized by neoplastic cells that express CD40.

A preferred agent within the context of the present invention is an agent based upon the binding determinants of the monoclonal antibody S2C6 (Paulie et al., (1984) Cancer Immunol. Immunother. 17:165-179). While S2C6 has been shown to have an agonist activity on human peripheral B cells as demonstrated by its ability to stimulate primary B cell proliferation in a dose dependent manner (Paulie et al., (1989) J. Immunol. 142:590-595), agents based upon the antibody have been shown to have anti-neoplastic activity in vivo (International Publication No. WO 00/75348).

CD40 specific agents included within the scope of the present invention include antibodies, optionally conjugated with or fused to a cytotoxic agent. Preferred CD40 specific agents are antibodies directed against CD40 such as those described above, preferable monoclonal antibodies or fragments thereof. In aspects of the invention where the antibody binds to the CD40 surface antigen and causes depletion of the CD40 bearing cell types, binding is generally characterized by being capable of homing to the CD40 antigen bearing cell type in vivo. Suitable binding agents bind the CD40 antigen with sufficient affinity and/or avidity such that the CD40 specific agent is useful as a therapeutic agent for targeting a cell expressing the antigen.

According to the present invention, an agent which arrests the growth of or causes the deletion of cells expressing CD40 is administered in combination with an agent which arrests the growth of or causes the deletion of cells expressing CD20. Such an agent, also referred to herein as a "CD20 specific agent" is a any molecule which inhibits the growth of or destroys or depletes CD20 expressing cells in a mammal. Appropriate CD20 specific agents may act through various mechanisms and the invention is not intended to be limited by the mechanism of action of the CD40 specific agent. Suitable CD20 specific agents may cause the depletion of cells expressing CD20 via mechanisms such as antibody mediated cellular cytotoxicity (ADCC) and/or complement dependent cytotoxicity (CDC), inhibition of B cell proliferation and/or induction of B cell death (e.g. via apoptosis).

CD20 specific agents included within the scope of the present invention include antibodies, optionally conjugated with or fused to a cytotoxic agent. Preferred CD20 specific agents are antibodies directed against CD20 preferably monoclonal antibodies or fragments thereof. In aspects of the invention where the antibody binds to the CD20 surface antigen and causes depletion of the CD20 bearing cell types, binding is generally characterized by being capable of located and homing to the CD20 antigen bearing cell type in vivo. Suitable binding agents bind the CD20 antigen with sufficient affinity and/or avidity such that the CD20 specific agent is useful as a therapeutic agent for targeting a cell expressing the antigen. Examples of antibodies which bind the CD20 antigen include: "C2B8" which is called "rituximab" (for example the "RITUXAN®" brand) (US Patent No. 5, 736, 137); the yttrium-[90]-labeled 2B8 murine antibody designated "Y2B8" (US Patent No. 5,736,137); murine IgG2a "B1" optionally labeled with ¹³¹I to generate the "¹³¹I-B1" antibody (BEXXAR™) (US Patent No. 5,595,721,); murine monoclonal antibody "1F5" (Press et al. Blood 69(2):584-591 (1987)); "chimeric 2H7" antibody (US Patent No. 5,677,180); and monoclonal antibodies L27, G28-2, 93-1B3, B-C1 or NU-B2 available from the International Leukocyte Typing Workshop (Valentine et al., In: Leukocyte Typing III (McMichael, Ed., p. 440, Oxford University Press (1987)).

The agents of the present invention directed against the CD40 and the CD20 surface antigens my be administered in combination with other growth arresting agents. Examples of growth inhibitory agents that do not bind the CD40 or CD20 membrane antigen include agents that block cell cycle progression (at a place other than S phase), such as agents that induce G1 arrest and M-phase arrest. Classical M-phase blockers include the vincas (vincristine and vinblastine), TAXOL®, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13.

In a preferred embodiment the agents which arrests the growth of, destroys or causes the deletion of cells expressing CD40 and CD20 are antibodies. A description follows as to exemplary techniques for the production of the preferred antibodies used in accordance with the present invention.

The CD40 or CD20 antigen used for production of antibodies may be, e.g., a soluble form of the extracellular domain of CD40 or CD20 or a portion thereof, containing the desired epitope. Alternatively, cells expressing CD40 or CD20 at their cell surface can be used to generate antibodies. Other forms of antigen useful for generating antibodies will be apparent to those skilled in the art.

### (i) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOC12, or R1N=C=NR, where R and R1 are different alkyl groups.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (ii) Monoclonal antibodies

Monoclonal antibodies are obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional antibody purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as E. coli cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs., 130:151-188 (1992).

In a further embodiment, monoclonal antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy chain and light chain constant domains in place of the homologous murine sequences (U.S. Patent No. 4,816,567; and Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iii) Humanized antibodies

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

An exemplary humanized antibody of interest herein is based upon the sequence of murine antibody S2C6 which recognizes CD40 and is described in International Publication Number WO 00/75348 and comprises variable heavy domain complementarity determining residues GYSFTGYYIH, (SEQ ID NO: 1), RVIPNNGGTSYNQKFKG (SEQ ID NO:2) and/or EGI---YW (SEQ ID NO:3), and optionally comprises amino acid modifications of those CDR residues, e.g. where the modifications essentially maintain or improve affinity of the antibody. For example, the antibody variant of interest may have from about one to about seven or about five amino acid substitutions in the above variable heavy CDR sequences.

The humanized antibody may comprise variable light domain complementarity determining residues from murine S2C6 as well. Therefore, light chain CDR residues RSSQSLVHSNGNTFLH (SEQ ID NO:4), TVSNRFS(SEQ ID NO:5); and SQTTHVPWT (SEQ ID NO:6), e.g. in addition to those variable heavy domain CDR residues in the preceding paragraph are preferred. Such humanized antibodies optionally comprise amino acid modifications of the above CDR residues, e.g. where the modifications essentially maintain or improve affinity of the antibody. For example, the antibody variant of interest may have from about one to about seven amino acid substitutions in the above variable light CDR sequences.

Various forms of the humanized or affinity matured antibody are contemplated. For example, the humanized or affinity matured antibody may be an antibody fragment, such as a Fab, which is optionally conjugated with one or more cytotoxic agent(s) in order to generate an immunoconjugate. Alternatively, the humanized or affinity matured antibody may be an intact antibody, such as an intact IgG1 antibody.

### (iv) Human antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggermann et al., Year in Immuno., 7:33 (1993); and U.S. Patent Nos. 5,591,669, 5,589,369 and 5,545,807.

Alternatively, phage display technology (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats; for their review see, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905. (v) Antibody fragments

Various techniques have been developed for the production of antibody fragments. Traditionally, these fragments were derived via proteolytic digestion of intact antibodies (see, e.g., Morimoto et al. , Journal of Biochemical and Biophysical Methods 24:107-117 (1992); and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form F(ab')2 fragments (Carter et al., Bio/Technology 10:163-167 (1992)). According to another approach, F(ab')2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; U.S. Patent No. 5,571,894; and U.S. Patent No. 5,587,458. The antibody fragment may also be a "linear antibody", e.g., as described in U.S. Patent 5,641,870 for example. Such linear antibody fragments may be monospecific or bispecific.

### (vi) Bispecific antibodies

Bispecific antibodies are antibodies that have binding specificities for at least two different epitopes. Exemplary bispecific antibodies may bind to two different epitopes of CD40 or CD20. Other such antibodies may combine a CD40 binding site with binding site(s) or CD20 or other tumor cell marker. Alternatively, an anti-CD20 or anti-CD40 arm may be combined with an arm which binds to a triggering molecule on a leukocyte such as a T-cell receptor molecule (e.g. CD2 or CD3), or Fc receptors for IgG (Fc R), such as Fc RI (CD64), Fc RII (CD32) and Fc RIII (CD16) so as to focus cellular defense mechanisms to the CD40 or CD20-expressing cell. Bispecific antibodies may also be used to localize cytotoxic agents to cells which express CD40 or CD20. These antibodies possess an CD20 or CD40-binding arm and an arm which binds the cytotoxic agent (e.g. saporin, anti-interferon-α, vinca alkaloid, ricin A chain, methotrexate or radioactive isotope hapten). Bispecific antibodies can be prepared as full length antibodies or antibody fragments (e.g. F(ab')2 bispecific antibodies).

Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the CH3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')2 fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

It may be desirable to modify the antibody of the invention with respect to effector function, e.g. so as to enhance antigen-dependent cell-mediated cyotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti-Cancer Drug Design 3:219-230 (1989).

To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (e.g., IgG1, IgG2, IgG3, or IgG4) that is responsible for increasing the in vivo serum half-life of the IgG molecule.

To identify antibodies within the context of the present invention directed against CD40 or CD20 such as growth inhibitory antibodies, one may screen for antibodies which inhibit the growth of cancer cells which express CD40 or CD20 such as the cell lines described in the Example sections herein.

### Disease or Disorder

The present invention provides a composition for the treatment of various diseases or disorders characterized by cells expressing CD40, especially those where any clinically desirable or beneficial effect, such as the regression, slowing or cessation of progression of the disease or disorder is achieved by arresting the growth of or deleting the CD40 expressing cells. Such diseases or disorders include neoplastic diseases or disorders including, but not limited to, benign and malignant tumors, including those of epithelial origin such as a carcinoma, mesodermal origin, such as a sarcoma and hematological malignancies, including leukemias, lymphomas and myelomas.

The disease or disorder of the present invention is typified by cells which expresses the CD40 surface antigen and cells which express the CD20 surface antigen. In the typical scenario the cell type that expresses the CD40 surface antigen expresses the CD20 surface antigen as well, although this is not a requirement. For example a disease or disorder characterized by cells expressing CD40 may be typified as well by cells expressing the CD20 surface antigen and these cells may be the same cells or different cells. Since the CD40 surface antigen expression precedes the expression of the CD20 antigen in development a subset of CD40 expressing cell types will express CD20 as well. On the other hand, cells which express CD20 typically also express CD40. Therefore a preferred disease or disorder characterized by cells expressing the CD40 surface antigen is any disease or disorder characterized by cells expressing the CD20 surface antigen.

Such diseases or disorders include various neoplastic diseases or disorders, including B cell malignancies. Non limiting examples of neoplastic diseases or disorders include those listed in Table I.

**Table I**

| |
|---|
| I. Hemotological Malignancies |
| A. Leukemia |
| acute leukemia |
| acute lymphocytic leukemia |
| acute myelocytic leukemia |
| myeloblastic |
| promyelocytic |
| myelomonocytic |
| monocytic |
| erythroleukemia |
| chronic leukemia |
| chronic myelocytic (granulocytic) leukemia |
| chronic lymphocytic leukemia |
| B. Polycythemia vera |
| C. Lymphoma |
| Hodgkin's disease |
| non-Hodgkin's disease |
| D. Multiple myeloma |
| E. Waldenström's macroglobulinemia |
| F. Heavy chain disease |
| II. Solid tumors |
| A. sarcomas and carcinomas |
| fibrosarcoma |
| myxosarcoma |
| liposarcoma |
| chondrosarcoma |
| osteogenic sarcoma |
| osteosarcoma |
| chordoma |
| angiosarcoma |
| endotheliosarcoma |
| lymphangiosarcoma |
| lymphangioendotheliosarcoma |
| synovioma |
| mesothelioma |
| Ewing's tumor |
| leiomyosarcoma |
| rhabdomyosarcoma |
| colon carcinoma |
| colorectal carcinoma |
| pancreatic cancer |
| breast cancer |
| ovarian cancer |
| prostate cancer |
| squamous cell carcinoma |
| basal cell carcinoma |
| adenocarcinoma |
| sweat gland carcinoma |
| sebaceous gland carcinoma |
| papillary carcinoma |
| papillary adenocarcinoma |
| cystadenocarcinoma |
| medullary carcinoma |
| bronchogenic carcinoma |
| renal cell carcinoma |
| hepatoma |
| bile duct carcinoma |
| choriocarcinoma |
| seminoma |
| embryonal carcinoma |
| Wilm's tumor |
| cervical cancer |
| uterine cancer |
| testicular tumor |
| lung carcinoma |
| small cell lung carcinoma |
| non small cell lung carcinoma |
| bladder carcinoma |
| epithelial carcinoma |
| glioma |
| astrocytoma |
| medulloblastoma |
| craniopharyngioma |
| ependymoma |
| pinealoma |
| hemangioblastoma |
| acoustic neuroma |
| oligodendroglioma |
| menangioma |
| melanoma |
| neuroblastoma |
| retinoblastoma |
| nasopharyngeal carcinoma |
| esophageal carcinoma |

Another class of diseases or disorders treatable within the context of the present invention are diseases or disorders of autoimmune aetiology such as those affecting a body tissue or organ, including skin, heart pericardium, endocardium, vasculature, a blood component (, e.g. erythrocytes, platelets), blood forming tissue (e.g. marrow, spleen), endocrine tissue or organs (e.g. pancreas, thyroid), gastrointestinal tract (e.g. bowel), respiratory tract (e.g. lung), kidney, central nervous system, peripheral nervous system, muscle and skeletal joints. Thus, the invention can be practiced in a subject with the symptoms, manifestations or risk factors for atopic dermatitis, any form of lupus (including cutaneous lupus (discoid lupus erythrematosus), and any extracutaneous type of lupus, including systemic lupus erythrematosus, acute lupus, lupus annularis, lupus discretus, lupus lymphaticus, lupus papollomatis, lupus psoriasis, lupus vulgaris, lupus sclerosis, neonatal lupus erythrematosus and drug-induced lupus), anti phospholipid syndrome, hemolytic anemia, idiopathic thrombocytopenia, thyroiditis, diabetes mellitus, inflammatory bowel disease, Crohn's disease, rhinitis, myasthenia gravis, rheumatoid arthritis and demyelinating diseases such as multiple sclerosis.

### Conjugates and Other Modifications of the Agents

The agents used in the methods or included in the articles of manufacture herein are optionally conjugated to a cytotoxic agent. Chemotherapeutic agents useful in the generation of such agent-cytotoxic agent conjugates have been described above. Conjugates of an agent and one or more small molecule toxins, such as a calicheamicin, a maytansine (US Patent No. 5,208,020), a trichothene, and CC1065 are also contemplated herein. In one embodiment of the invention, the agent is conjugated to one or more maytansine molecules (e.g. about 1 to about 10 maytansine molecules per agent molecule). Maytansine may, for example, be converted to May-SS-Me which may be reduced to May-SH3 and reacted with modified agent (Chari et al. Cancer Research 52: 127-131 (1992)) to generate a maytansinoid-agent conjugate.

Alternatively, the agent is conjugated to one or more calicheamicin molecules. The calicheamicin family of antibiotics are capable of producing double-stranded DNA breaks at sub-picomolar concentrations. Structural analogues of calicheamicin which may be used include, but are not limited to, 1I, 2I, 3I, N-acetyl- 1I, PSAG and I1 (Hinman et al. Cancer Research 53: 3336-3342 (1993) and Lode et al. Cancer Research 58: 2925-2928 (1998)).

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an agent conjugated with a compound with nucleolytic activity (e.g. a ribonuclease or a DNA endonuclease such as a deoxyribonuclease; DNase).

A variety of radioactive isotopes are available for the production of radioconjugated agents suitable within the context of the present invention. Examples include At211, I131, I125, Y90, Re186, Re188, Sm153, Bi212, P32 and radioactive isotopes of Lu.

Conjugates of an agent and cytotoxic agent may be made using a variety of bifunctional protein coupling agents such as N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the agent. See WO94/11026. The linker may be a "cleavable linker" facilitating release of the cytotoxic drug in the cell. For example, an acid-labile linker, peptidase-sensitive linker, dimethyl linker or disulfide-containing linker (Chari et al. Cancer Research 52: 127-131 (1992)) may be used.

Alternatively, a fusion protein comprising the agent and cytotoxic agent may be made, e.g. by recombinant techniques or peptide synthesis.

Single chain immunotoxins may be used. Single chain immunotoxins are bifunctional molecules consisting of an antibody binding domain fused to a protein toxin. Once bound to the target cells, immunotoxins internalize into endocytic vesicles where the catalytic portion of the toxin is processed and released into the cytosol. Once in the cytosol, protein syntheses is halted and cell death ensues.

### Formulations

Formulations of the active agents within the context of the present invention will vary and depend upon several factors known to the skilled artisan including the route of administration and the active agent, which is an antibody. The skilled artisan will recognize that antibodies used in accordance with the present invention may be prepared for storage and later administration by combining an antibody having the desired degree of purity with optional pharmaceutically acceptable carriers, excipients or stabilizers (Remington's Pharmaceutical Sciences 16th edition, Osol, A. Ed. (1980)), often in the form of lyophilized powders or as aqueous solutions. Suitable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The active ingredients may also be entrapped in microcapsules, microcapsules, in colloidal drug delivery systems, liposomes, albumin microspheres, microemulsions, nano-particles and nanocapsules or in macroemulsions. Sustained-release preparations may be prepared in accordance with art recognized procedures. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides, copolymers of L-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37 C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### Treatment

It is contemplated that the agents including antibodies of the invention are administered to a subject in need thereof, in accord with known methods, such as intravenous administration as a bolus or by continuous infusion over a period of time, by intramuscular, intraperitoneal, intracerebrospinal, subcutaneous, intra-articular, intrasynovial, intrathecal, oral, topical, or inhalation routes. Intravenous or subcutaneous administration of the antibody is preferred. Typically, maintenance doses are delivered by bolus delivery, preferably by subcutaneous bolus administration, making treatment convenient and cost-effective for the patient and health care professionals. The CD40 specific agent and the CD20 specific agent may be administered simultaneously or separately, one specific agent following the other.

Where combined administration of a chemotherapeutic agent is desired, the combined administration includes coadministration, using separate formulations, and consecutive administration in either order, wherein preferably there is a time period while both (or all) active agents simultaneously exert their biological activities. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the antibody or may be given simultaneously therewith. Exemplary antibody formulations are described in WO98/56418. This publication describes a liquid multidose formulation comprising 40 mg/mL rituximab, 25 mM acetate, 150 mM trehalose, 0.9% benzyl alcohol, 0.02% polysorbate 20 at pH 5.0 that has a minimum shelf life of two years storage at 2-8 C. Another anti-CD20 formulation of interest comprises 10mg/mL rituximab in 9.0 mg/mL sodium chloride, 7.35 mg/mL sodium citrate dihydrate, 0.7mg/mL polysorbate 80, and Sterile Water for Injection, pH 6.5.

It may be desirable to also administer antibodies against other tumor associated antigens, such as antibodies which bind to the EGFR, ErbB3, ErbB4, or vascular endothelial growth factor (VEGF). Alternatively, or additionally, two or more anti-CD40 antibodies may be co-administered to the patient. Sometimes, it may be beneficial to also administer one or more cytokines to the patient. The antibody may be co-administered with a growth inhibitory agent. For example, the growth inhibitory agent may be administered first, followed by the active agent such as an antibody. However, simultaneous administration, or administration of the antibody first is also contemplated. Suitable dosages for the growth inhibitory agent are those presently used and may be lowered due to the combined action (synergy) of the growth inhibitory agent and the active agent.

In addition to the above therapeutic regimens, the patient may be subjected to surgical removal of cancer cells and/or radiation therapy.

For the prevention or treatment of disease, the appropriate dosage of agent including antibodies will depend on the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Where the treatment involves a series of treatments, the initial dose or initial doses are followed at daily or weekly intervals by maintenance doses. Each maintenance dose provides the same or a smaller amount of antibody compared to the amount of antibody administered in the initial dose or doses.

Depending on the type and severity of the disease, about 1 µg/kg to 15 mg/kg (e.g. 0.1-20mg/kg) of antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. The progress of this therapy is easily monitored by conventional techniques and assays.

### Articles of Manufacture

In another embodiment of the invention, an article of manufacture containing materials useful for the treatment of the disorders described above is provided. The article of manufacture comprises a container, a label and a package insert. Suitable containers include, for example, bottles, vials, syringes, etc. The containers may be formed from a variety of materials such as glass or plastic. The container holds a composition which is effective for treating the condition and may have a sterile access port (for example, the container may be an intravenous solution bag or a vial having a stopper pierceable by a hypodermic injection needle). At least one active agent in the composition is an anti-CD40 antibody. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, and syringes.

The following examples are offered by way of illustration and not by way of limitation.

### EXAMPLES

### EXAMPLE I

### Materials and Methods

### Animal

Female CB-17 IcrTac-scidfDF mice (8-9 weeks old) were obtained from Taconic (Germantown, NY 12526).

### Tumor Cell lines

Ramos EBV-negative Burkitt's lymphoma, HS Sultan EBV-positive plasmacytoma and IM9 EBV-positive multiple myeloma cell lines were purchased from American Type Culture Collection (Manassas, VA 20110). Rituxan resistant Ramos lymphoma cell line was established through exposing the Ramos lymphoma cell line to high doses of Rituxan (500ug/mouse IP, 3 times/week for 3 weeks) in a subcutaneous xenograft scid mouse.

### Dissemination tumor model

Mice were injected through the tail vein with 1x10e6 tumor cells in 100 ul HBSS. Treatment with control antibody or SGN-14 or Rituxan or SGN-14 and Rituxan combination or chimeric SGN-14 mAb or chimeric SGN-14 mAb and Rituxan or human anti-CD40 mAb was started on day 3 after tumor inoculation. The antibodies were given intraperitoneally 100ug per mouse in 100ul sterile saline at the frequency of 3 times a week for a total of 3 weeks treatment. The mice were monitored twice daily for mortality. The cause of death was confirmed by histopathological evaluation.

### Subcutaneous tumor model

Mice were injected subcutaneously at the right flank with 5x10e6 tumor cells in 100 ul HBSS. Treatment began when the tumor volume reached ~150 cubic mm with either control antibody or SGN-14 or Rituxan or SGN-14 and Rituxan combination or chimeric SGN-14 mAb or chimeric SGN-14 mAb and Rituxan or human anti-CD40 mAb. Each mouse received 100ug of one of the antibody in 100ul sterile saline intraperitoneally 3 times a week for a total of 3 weeks treatment. The tumor volume was measured weekly.

### Results

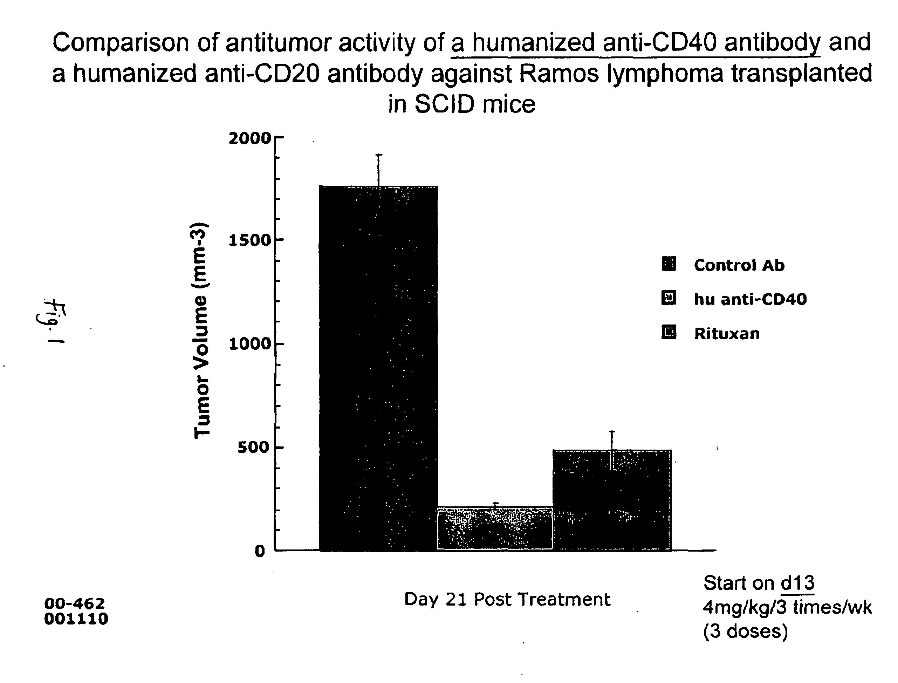

Comparison was made of antitumor activity of a humanized anti-CD40 antibody based upon the murine CDRs of S2C6 (International Publication No. WO 00/75348) and an anti-CD20 antibody (RITUXAN® brand product) against Ramos lymphoma transplanted in SCID mice. Results are presented in Figure 1. At 21 days post treatment tumor volume was significantly reduced compared with control in animals receiving humanized anti-CD40 antibody and in animals receiving anti-CD20 antibody.

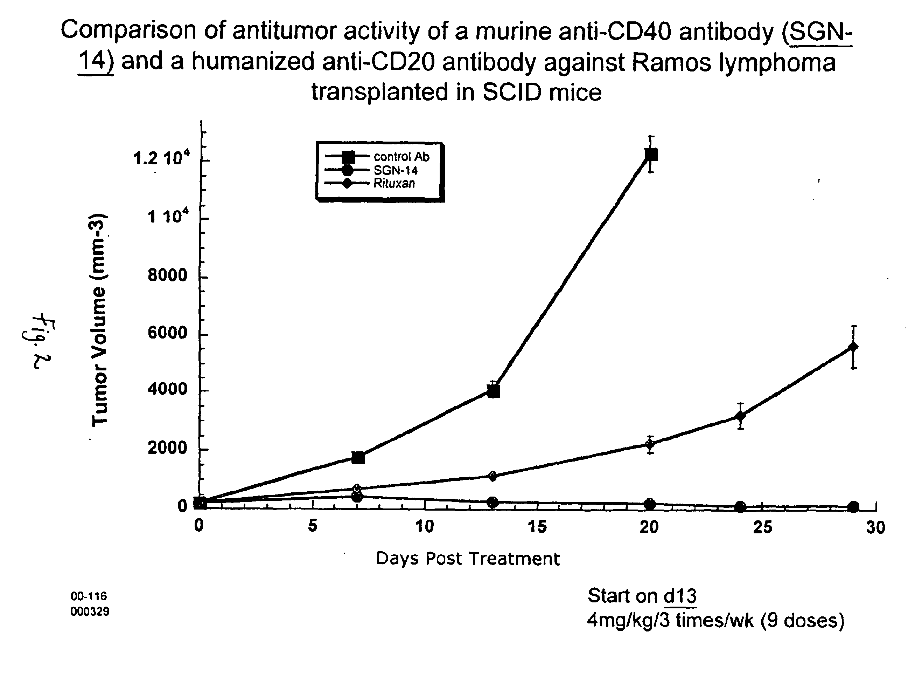

Comparison was made of antitumor activity of a murine anti-CD40 antibody (S2C6 (SGN14) International Publication No. WO 00/75348) and an anti-CD20 antibody (RITUXAN® brand product) against Ramos lymphoma transplanted in SCID mice. Results are presented in Figure 2. Tumor volume at 31 days post treatment was significantly reduced in animals receiving anti-CD40 antibody compared with control as well as with animals receiving anti-CD20 antibody.

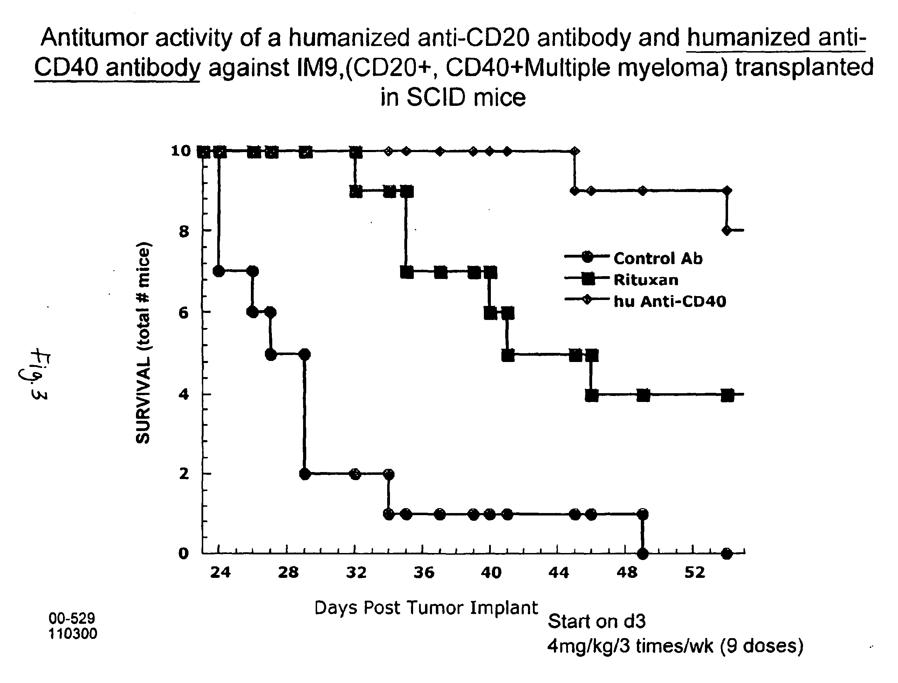

Antitumor activity of an anti-CD20 antibody (RITUXAN® brand product) and a humanized anti-CD40 antibody (based upon the CDR's of murine S2C6 (SGN-14) International Publication No. WO 00/75348) against IM9, (CD20+, CD40+ Multiple myeloma) was assessed in transplanted SCID mice. Results are presented in Figure 3. Survival post implant was extended in animals receiving anti-CD40 antibody compared with both control and animals receiving anti-CD20 antibody.

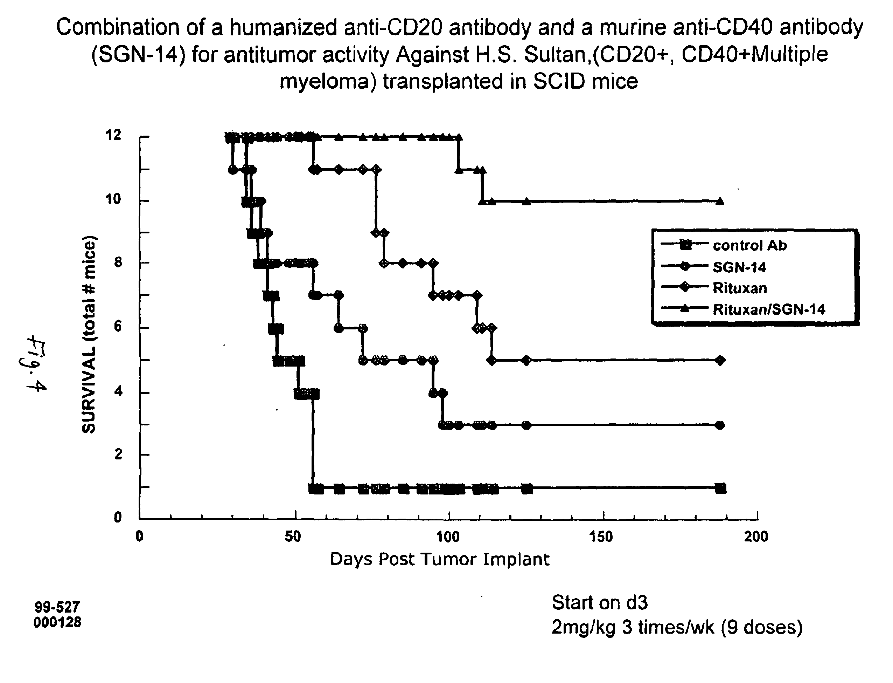

The combination of an anti-CD20 antibody (RITUXAN® brand product) and a murine anti-CD40 antibody (S2C6 (SGN-14) International Publication No. WO 00/75348) was assessed for antitumor activity against H.S. Sultan, (CD20+, CD40+Multiple myeloma) transplanted in SCID mice. The results are presented in Figure 4. Survival was extended in mice receiving a combination of anti-CD40 antibody and anti-CD20 antibody compared with control animals and animals receiving anti-CD40 antibody or anti-CD20 antibody alone.

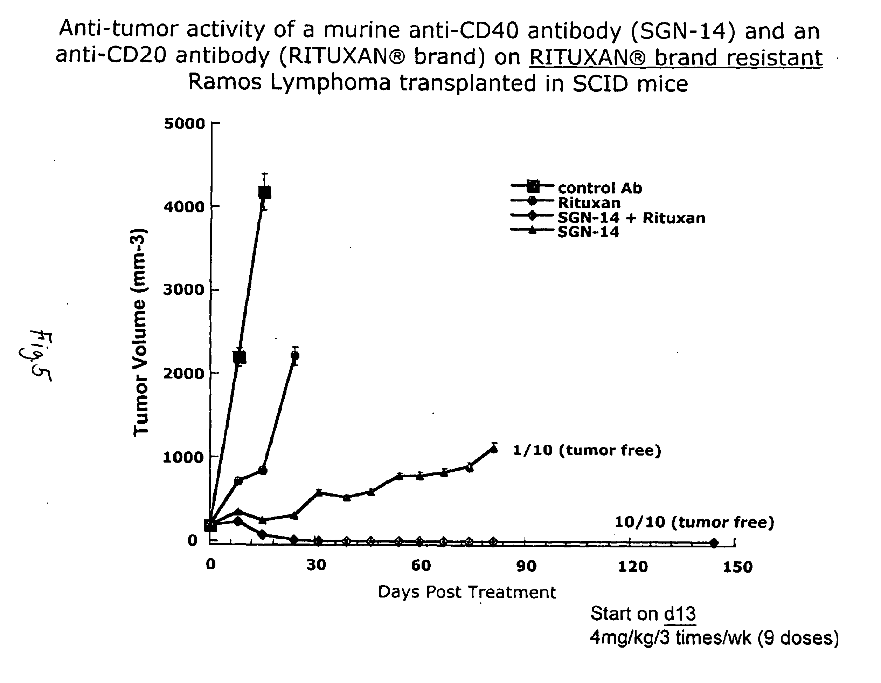

Anti-tumor activity of a murine anti-CD40 antibody (S2C6 (SGN-14) International Publication No. WO 00/75348) and an anti-CD20 antibody (RITUXAN® brand product) on RITUXAN® brand resistant Ramos lymphoma in transplanted SCID mice. The results are presented in Figure 5. Tumor volume in mice receiving a combination of anti-CD40 antibody and an anti-CD20 antibody was significantly reduced compared with each of control animals and animals receiving anti-CD20 or anti-CD40 antibody alone.

## Claims

1. A composition consisting of an antibody directed against CD40, plus optional pharmaceutically acceptable carriers, pharmaceutical excipients or pharmaceutical stabilisers, wherein the antibody arrests the growth of or causes deletion of cells expressing CD40 and is optionally conjugated to a radioactive isotope, chemotherapeutic agent, toxin or fragment thereof,
for use in treatment of a disease or disorder **characterised by** cells expressing CD40 in a mammal,
wherein the treatment comprises administration of said composition in combination with a second composition, wherein the second composition consists of an antibody directed against CD20 plus optional pharmaceutically acceptable carriers, pharmaceutical excipients or pharmaceutical stabilisers, and wherein the antibody directed against CD20 arrests the growth of or causes deletion of cells expressing CD20 and is optionally conjugated to a radioactive isotope,
chemotherapeutic agent, toxin or fragment thereof.

2. Use of an antibody directed against CD40, wherein the antibody arrests the growth of or causes deletion of cells expressing CD40, for the manufacture of a medicament for treatment of a disease or disorder **characterised by** cells expressing CD40 in a mammal, wherein
the medicament consists of the antibody directed against CD40 optionally conjugated to a radioactive isotope, chemotherapeutic agent, toxin or fragment thereof, and optional pharmaceutically acceptable carriers, pharmaceutical excipients or pharmaceutical stabilisers, and wherein
the treatment comprises administration of the medicament in combination, with a composition consisting of an antibody directed against CD20, optionally conjugated to a radioactive isotope, chemotherapeutic agent, toxin or fragment thereof, and optional pharmaceutically acceptable carriers, pharmaceutical excipients or pharmaceutical stabililsers, wherein the antibody directed against CD20 arrests the growth of or causes deletion of cells expressing CD20.

3. A composition consisting of an antibody directed against CD20 plus optional pharmaceutically acceptable carriers, pharmaceutical excipients or pharmaceutical stabilisers, wherein the antibody arrests the growth of or causes deletion of cells expressing CD20 and is optionally conjugated to a radioactive isotope chemotherapeutic agent, toxin or fragment thereof,
for use in treatment of a disease or disorder **characterised by** cells expressing CD40 in a mammal,
wherein the treatment comprises administration of said composition in combination with a second composition, wherein the second composition consists of an antibody directed against CD40 plus optional pharmaceutically acceptable carriers, pharmaceutical excipients or pharmaceutical stabilisers, wherein the antibody directed against CD40 arrests the growth of or causes deletion of cells expressing CD40 and is optionally conjugated to a radioactive isotope, chemotherapeutic agent, toxin or fragment thereof.

4. Use of an antibody directed against CD20, wherein the antibody arrests the growth of or causes deletion of cells expressing CD20, for the manufacture of a medicament for treatment of a disease or disorder **characterised by** cells expressing CD40 in a mammal, wherein
the medicament, consists of the antibody directed against CD20, optionally conjugated to a radioactive isotope, chemotherapeutic agent, toxin or fragment thereof, and optional pharmaceutically acceptable carriers, pharmaceutical excipients or pharmaceutical stabilisers, and wherein
the treatment comprises administration of the medicament in combination with a composition consisting of an antibody directed against CD40 and optional pharmaceutically acceptable carriers, pharmaceutical excipients or pharmaceutical stabilisers, wherein the antibody directed against CD40 arrests the growth of or causes deletion of cells expressing CD40 and is optionally conjugated to a radioactive isotope, chemotherapeutic agent, toxin or fragment thereof.

5. A composition or use according to any of the preceding claims, wherein the treatment comprises administering the antibody directed against CD20 and the antibody directed against CD40 separately, one following the other.

6. A omposition or use according to any of the preceding claims, wherein the disease or disorder is a neoplastic disease or disorder.

7. A composition for use or use according to claim 6, wherein the treatment further comprises administration of one or more chemotherapeutic agents in a separate formulation or formulations.

8. A composition for use or use according to claim 7, wherein the treatment comprises simultaneous administration of the chemotherapeutic agent with the antibody directed against CD40 and/or the antibody directed against CD20.

9. A composition for use or use according to claim 7 or claim 8, wherein the one or more chemotherapeutic agents comprise gemcitabine.

10. A composition for use according to any of claims 7 to 9 wherein the one or more chemotherapeutic agents comprise ifosfamide, carboplatin and etoposide.

11. A composition for use or use according to any of claims 6 to 10, wherein the neoplastic disease or disorder is a hematological malignancy.

12. A composition for use or use according to any of claims 5 to 10, wherein the neoplastic disease or disorder is a solid tumor.

13. A composition for use or use according to claim 11 wherein the malignancy is a lymphoma.

14. A composition for use or use according to claim 13 wherein the lymphoma is a non-hodgkins type lymphoma.

15. A composition for use or use according to claim 11 wherein the malignancy is a myeloma.

16. A composition for use or use according to claim 15 wherein the myeloma is a multiple myeloma.

17. A composition for use or use according to claim 11 wherein the malignancy is a leukemia.

18. A composition for use or use according to claim 17, wherein the leukemia is chronic lymphocytic leukemia.

19. A composition for use or use according to any of the preceding claims, wherein the disease or disorder is an autoimmune disease or disorder.

20. A composition for use or use according to claim 19, wherein the autoimmune disease is rheumatoid arthritis.

21. A composition for use or use according to claim 19, wherein the autoimmune disease is systemic lupus erythrematosus.

22. A composition for use or use according to any of the preceding claims wherein the antibody directed against CD40 is a monoclonal antibody.

23. A composition for use or use according to claim 22, wherein the monoclonal antibody directed against CD40 is a chimeric antibody.

24. A composition for use or use according to claim 23, wherein the antibody directed against CD40 is a humanized antibody.

25. A composition for use or use according to claim 22, wherein the antibody directed against CD40 competes for binding of CD40 with the monoclonal antibody S2C6.

26. A composition for use or use according to claim 22, wherein the antibody directed against CD40 has the binding characteristics of monoclonal antibody S2C6.

27. A composition for use or use according to any of the preceding claims, wherein the antibody directed against CD20 is a monoclonal antibody.

28. A composition for use or use according to claim 27, wherein the monoclonal antibody directed against CD20 is a chimeric antibody.

29. A composition for use or use according to claim 28 wherein the monoclonal antibody directed against CD20 is C2B8.

## Patentansprüche

1. Zusammensetzung, die aus einem gegen CD40 gerichteten Antikörper und optionalen pharmazeutisch annehmbaren Trägern, pharmazeutischen Exzipienten oder pharmazeutischen Stabilisatoren besteht, worin der Antikörper das Wachstum von Zellen, die CD40 exprimieren, aufhält oder die Zerstörung von Zellen, die CD40 exprimieren, verursacht und gegebenenfalls an ein radioaktives Isotop, Chemotherapeutikum, Toxin oder Fragment davon konjugiert ist,
zur Verwendung bei der Behandlung einer Krankheit oder Störung, die durch Zellen, die CD40 exprimieren, **gekennzeichnet** ist, in einem Säugetier,
worin die Behandlung die Verabreichung der Zusammensetzung in Kombination mit einer zweiten Zusammensetzung umfasst, worin die zweite Zusammensetzung aus einem gegen CD20 gerichteten Antikörper und optionalen pharmazeutisch annehmbaren Trägern, pharmazeutischen Exzipienten oder pharmazeutischen Stabilisatoren besteht und worin der gegen CD20 gerichtete Antikörper das Wachstum von Zellen, die CD20 exprimieren, aufhält oder die Zerstörung von Zellen, die CD20 exprimieren, verursacht und gegebenenfalls an ein radioaktives Isotop, Chemotherapeutikum, Toxin oder Fragment davon konjugiert ist.

2. Verwendung eines gegen CD40 gerichteten Antikörpers, worin der Antikörper das Wachstum von Zellen, die CD 40 exprimieren, aufhält oder die Zerstörung von Zellen, die CD40 exprimieren, verursacht, zur Herstellung eines Medikaments zur Behandlung einer Krankheit oder Störung, die durch Zellen, die CD40 exprimieren, **gekennzeichnet** ist, in einem Säugetier, worin
das Medikament aus dem gegen CD40 gerichteten Antikörper, der gegebenenfalls an ein radioaktives Isotop, Chemotherapeutikum, Toxin oder Fragment davon konjugiert ist, und optionalen pharmazeutisch annehmbaren Trägern, pharmazeutischen Exzipienten oder pharmazeutischen Stabilisatoren besteht und worin
die Behandlung die Verabreichung des Medikaments in Kombination mit einer Zusammensetzung, die aus einem gegen CD20 gerichteten Antikörper, der gegebenenfalls an ein radioaktives Isotop, Chemotherapeutikum, Toxin oder Fragment davon konjugiert ist, und optionalen pharmazeutisch annehmbaren Trägern, pharmazeutischen Exzipienten oder pharmazeutischen Stabilisatoren besteht, umfasst, worin der gegen CD20 gerichtete Antikörper das Wachstum von Zellen, die CD20 exprimieren, aufhält oder die Zerstörung von Zellen, die CD20 exprimieren, verursacht.

3. Zusammensetzung, die aus einem gegen CD20 gerichteten Antikörper und optionalen pharmazeutisch annehmbaren Trägern, pharmazeutischen Exzipienten oder pharmazeutischen Stabilisatoren besteht, worin der Antikörper das Wachstum von Zellen, die CD20 exprimieren, aufhält oder die Zerstörung von Zellen, die CD20 exprimieren, verursacht und gegebenenfalls an ein radioaktives Isotop, Chemotherapeutikum, Toxin oder Fragment davon konjugiert ist,
zur Verwendung bei der Behandlung einer Krankheit oder Störung, die durch Zellen, die CD40 exprimieren, **gekennzeichnet** ist, in einem Säugetier,
worin die Behandlung die Verabreichung der Zusammensetzung in Kombination mit einer zweiten Zusammensetzung umfasst, worin die zweite Zusammensetzung aus einem gegen CD40 gerichteten Antikörper und optionalen pharmazeutisch annehmbaren Trägern, pharmazeutischen Exzipienten oder pharmazeutischen Stabilisatoren besteht, worin der gegen CD40 gerichtete Antikörper das Wachstum von Zellen, die CD40 exprimieren, aufhält oder die Zerstörung von Zellen, die CD40 exprimieren, verursacht und gegebenenfalls an ein radioaktives Isotop, Chemotherapeutikum, Toxin oder Fragment davon konjugiert ist.

4. Verwendung eines gegen CD20 gerichteten Antikörpers, worin der Antikörper das Wachstum von Zellen, die CD 20 exprimieren, aufhält oder die Zerstörung von Zellen, die CD20 exprimieren, verursacht, zur Herstellung eines Medikaments zur Behandlung einer Krankheit oder Störung, die durch Zellen, die CD40 exprimieren, **gekennzeichnet** ist, in einem Säugetier, worin
das Medikament aus dem gegen CD20 gerichteten Antikörper, der gegebenenfalls an ein radioaktives Isotop, Chemotherapeutikum, Toxin oder Fragment davon konjugiert ist, und optionalen pharmazeutisch annehmbaren Trägern, pharmazeutischen Exzipienten oder pharmazeutischen Stabilisatoren besteht und worin
die Behandlung die Verabreichung des Medikaments in Kombination mit einer Zusammensetzung, die aus einem gegen CD40 gerichteten Antikörper und optionalen pharmazeutisch annehmbaren Trägern, pharmazeutischen Exzipienten oder pharmazeutischen Stabilisatoren besteht, umfasst, worin der gegen CD40 gerichtete Antikörper das Wachstum von Zellen, die CD40 exprimieren, aufhält oder die Zerstörung von Zellen, die CD40 exprimieren, verursacht und gegebenenfalls an ein radioaktives Isotop, Chemotherapeutikum, Toxin oder Fragment davon konjugiert ist.

5. Zusammensetzung oder Verwendung nach einem der vorangegangenen Ansprüche, worin die Behandlung das getrennte Verabreichen des gegen CD20 gerichteten Antikörpers und des gegen CD40 gerichteten Antikörpers nacheinander umfasst.

6. Zusammensetzung zur Verwendung oder Verwendung nach einem der vorangegangenen Ansprüche, worin die Krankheit oder Störung eine neoplastische Krankheit oder Störung ist.

7. Zusammensetzung zur Verwendung oder Verwendung nach Anspruch 6, worin die Behandlung weiters die Verabreichung von einem oder mehreren Chemotherapeutikum/Chemotherapeutika in einer getrennten Formulierung oder in getrennten Formulierungen umfasst.

8. Zusammensetzung zur Verwendung oder Verwendung nach Anspruch 7, worin die Behandlung die gleichzeitige Verabreichung des Chemotherapeutikums mit dem gegen CD40 gerichteten Antikörper und/oder dem gegen CD20 gerichteten Antikörper umfasst.

9. Zusammensetzung zur Verwendung oder Verwendung nach Anspruch 7 oder Anspruch 8, worin das eine Chemotherapeutikum oder die mehreren Chemotherapeutika Gemcitabin umfasst/umfassen.

10. Zusammensetzung zur Verwendung oder Verwendung nach einem der Ansprüche 7 bis 9, worin das eine Chemotherapeutikum oder die mehreren Chemotherapeutika Ifosfamid, Carboplatin und Etoposid umfasst/umfassen.

11. Zusammensetzung zur Verwendung oder Verwendung nach einem der Ansprüche 6 bis 10, worin die neoplastische Krankheit oder Störung eine hämatologische Malignität ist.

12. Zusammensetzung zur Verwendung oder Verwendung nach einem der Ansprüche 5 bis 10, worin die neoplastische Erkrankung oder Störung ein fester Tumor ist.

13. Zusammensetzung zur Verwendung oder Verwendung nach Anspruch 11, worin die Malignität ein Lymphom ist.

14. Zusammensetzung zur Verwendung oder Verwendung nach Anspruch 13, worin das Lymphom ein Nicht-Hodgkin-artiges Lymphom ist.

15. Zusammensetzung zur Verwendung oder Verwendung nach Anspruch 11, worin die Malignität ein Myelom ist.

16. Zusammensetzung zur Verwendung oder Verwendung nach Anspruch 15, worin das Myelom ein multiples Myelom ist.

17. Zusammensetzung zur Verwendung oder Verwendung nach Anspruch 11, worin die Malignität Leukämie ist.

18. Zusammensetzung zur Verwendung oder Verwendung nach Anspruch 17, worin die Leukämie eine chronische lymphozytische Leukämie ist.

19. Zusammensetzung zur Verwendung oder Verwendung nach einem der vorangegangenen Ansprüche, worin die Erkrankung oder Störung eine Autoimmunkrankheit oder -störung ist.

20. Zusammensetzung zur Verwendung oder Verwendung nach Anspruch 19, worin die Autoimmunstörung rheumatoide Arthritis ist.

21. Zusammensetzung zur Verwendung oder Verwendung nach Anspruch 19, worin die Autoimmunkrankheit systemischer Lupus erythematodes ist.

22. Zusammensetzung zur Verwendung oder Verwendung nach einem der vorangegangenen Ansprüche, worin der gegen CD40 gerichtete Antikörper ein monoklonaler Antikörper ist.

23. Zusammensetzung zur Verwendung oder Verwendung nach Anspruch 22, worin der gegen CD40 gerichtete monoklonale Antikörper ein chimärer Antikörper ist.

24. Zusammensetzung zur Verwendung oder Verwendung nach Anspruch 23, worin der gegen CD40 gerichtete Antikörper ein humanisierter Antikörper ist.

25. Zusammensetzung zur Verwendung oder Verwendung nach Anspruch 22, worin der gegen CD40 gerichtete Antikörper um die Bindung des CD40 mit dem monoklonalen Antikörper S2C6 konkurriert.

26. Zusammensetzung zur Verwendung oder Verwendung nach Anspruch 22, worin der gegen CD40 gerichtete Antikörper die Bindungseigenschaften des monoklonalen Antikörpers S2C6 aufweist.

27. Zusammensetzung zur Verwendung oder Verwendung nach einem der vorangegangenen Ansprüche, worin der gegen CD20 gerichtete Antikörper ein monoklonaler Antikörper ist.

28. Zusammensetzung zur Verwendung oder Verwendung nach Anspruch 27, worin der gegen CD20 gerichtete monoklonale Antikörper ein chimärer Antikörper ist.

29. Zusammensetzung zur Verwendung oder Verwendung nach Anspruch 28, worin der gegen CD20 gerichtete monoklonale Antikörper C2B8 ist.

## Revendications

1. Composition consistant en un anticorps dirigé contre CD40, plus des supports pharmaceutiquement acceptables, excipients pharmaceutiques ou stabilisants pharmaceutiques facultatifs, dans laquelle l'anticorps interrompt la croissance ou provoque la délétion de cellules exprimant CD40 et est éventuellement conjugué avec des isotopes radioactifs, des agents chimiothérapeutiques, des toxines ou leurs fragments,
destinée à être utilisée dans le traitement d'une maladie ou d'un trouble **caractérisé par** des cellules exprimant CD40 chez un mammifère,
le traitement comprenant l'administration de ladite composition en association avec une seconde composition, ladite seconde composition consistant en un anticorps dirigé contre CD20 plus des supports pharmaceutiquement acceptables, excipients pharmaceutiques ou stabilisants pharmaceutiques facultatifs, et l'anticorps dirigé contre CD20 interrompant la croissance ou provoquant la délétion de cellules exprimant CD20 et étant éventuellement conjugué avec des isotopes radioactifs, des agents chimiothérapeutiques, des toxines ou leurs fragments.

2. Utilisation d'un anticorps dirigé contre CD40, dans laquelle l'anticorps interrompt la croissance ou provoque la délétion de cellules exprimant CD40, pour la production d'un médicament destiné au traitement d'une maladie ou d'un trouble **caractérisé par** des cellules exprimant CD40 chez un mammifère, où le médicament consiste en l'anticorps dirigé contre CD40, éventuellement conjugué avec des isotopes radioactifs, des agents chimiothérapeutiques, des toxines ou leurs fragments, et des supports pharmaceutiquement acceptables, excipients pharmaceutiques ou stabilisants pharmaceutiques facultatifs, et où
le traitement comprend l'administration du médicament en association avec une composition consistant en un anticorps dirigé contre CD20, éventuellement conjugué avec des isotopes radioactifs, des agents chimiothérapeutiques, des toxines ou leurs fragments, et des supports pharmaceutiquement acceptables, excipients pharmaceutiques ou stabilisants pharmaceutiques facultatifs, l'anticorps dirigé contre CD20 interrompant la croissance ou provoquant la délétion de cellules exprimant CD20.

3. Composition consistant en un anticorps dirigé contre CD20, plus des supports pharmaceutiquement acceptables, excipients pharmaceutiques ou stabilisants pharmaceutiques facultatifs, l'anticorps interrompant la croissance ou provoquant la délétion de cellules exprimant CD20 et étant éventuellement conjugué avec des isotopes radioactifs, des agents chimiothérapeutiques, des toxines ou leurs fragments,
destinée à être utilisée dans le traitement d'une maladie ou d'un trouble **caractérisé par** des cellules exprimant CD40 chez un mammifère,
le traitement comprenant l'administration de ladite composition en association avec une seconde composition, ladite seconde composition consistant en un anticorps dirigé contre CD40 plus des supports pharmaceutiquement acceptables, excipients pharmaceutiques ou stabilisants pharmaceutiques facultatifs, et l'anticorps dirigé contre CD40 interrompant la croissance ou provoquant la délétion de cellules exprimant CD40 et étant éventuellement conjugué avec des isotopes radioactifs, des agents chimiothérapeutiques, des toxines ou leurs fragments.

4. Utilisation d'un anticorps dirigé contre CD20, dans laquelle l'anticorps interrompt la croissance ou provoque la délétion de cellules exprimant CD20, pour la production d'un médicament destiné au traitement d'une maladie ou d'un trouble **caractérisé par** des cellules exprimant CD40 chez un mammifère, où
le médicament consiste en l'anticorps dirigé contre CD20, éventuellement conjugué avec des isotopes radioactifs, des agents chimiothérapeutiques, des toxines ou leurs fragments, et des supports pharmaceutiquement acceptables, excipients pharmaceutiques ou stabilisants pharmaceutiques facultatifs, et où
le traitement comprend l'administration du médicament en association avec une composition consistant en un anticorps dirigé contre CD40, et des supports pharmaceutiquement acceptables, excipients pharmaceutiques ou stabilisants pharmaceutiques facultatifs, l'anticorps dirigé contre CD40 interrompant la croissance ou provoquant la délétion de cellules exprimant CD40 et étant éventuellement conjugué avec des isotopes radioactifs, des agents chimiothérapeutiques, des toxines ou leurs fragments.

5. Composition ou utilisation suivant l'une quelconque des revendications précédentes, dans laquelle le traitement comprend l'administration de l'anticorps dirigé contre C20 et de l'anticorps dirigé contre CD40 séparément, l'un après l'autre.

6. Composition destinée à être utilisée ou utilisation suivant l'une quelconque des revendications précédentes, dans laquelle la maladie ou le trouble est une maladie ou un trouble néoplasique.

7. Composition destinée à être utilisée ou utilisation suivant la revendication 6, dans laquelle le traitement comprend en outre l'administration d'un ou plusieurs agents chimiothérapeutiques en une ou plusieurs formulations séparées.

8. Composition destinée à être utilisée ou utilisation suivant la revendication 7, dans laquelle le traitement comprend l'administration simultanée de l'agent chimio-thérapeutique avec l'anticorps dirigé contre CD40 et/ou l'anticorps dirigé contre CD20.

9. Composition destinée à être utilisée ou utilisation suivant la revendication 7 ou la revendication 8, dans laquelle le ou les agents chimiothérapeutiques comprennent la gemcitabine.

10. Composition destinée à être utilisée suivant l'une quelconque des revendications 7 à 9, dans laquelle le ou les agents chimiothérapeutiques comprennent l'ifosfamide, le carboplatine et l'étoposide.

11. Composition destinée à être utilisée ou utilisation suivant l'une quelconque des revendications 6 à 10, dans laquelle la maladie ou le trouble néoplasique est une affection maligne hématologique

12. Composition destinée à être utilisée ou utilisation suivant l'une quelconque des revendications 5 à 10, dans laquelle la maladie ou le trouble néoplasique est une tumeur solide.

13. Composition destinée à être utilisée ou utilisation suivant la revendication 11, dans laquelle l'affection maligne est un lymphome.

14. Composition destinée à être utilisée ou utilisation suivant la revendication 13, dans laquelle le lymphome est un lymphome de type non hodgkinien.

15. Composition destinée à être utilisée ou utilisation suivant la revendication 11, dans laquelle l'affection maligne est un myélome.

16. Composition destinée à être utilisée ou utilisation suivant la revendication 15, dans laquelle le myélome est un myélome multiple.

17. Composition destinée à être utilisée ou utilisation suivant la revendication 11, dans laquelle l'affection maligne est une leucémie.

18. Composition destinée à être utilisée ou utilisation suivant la revendication 17, dans laquelle la leucémie est la leucémie lymphocytaire chronique.

19. Composition destinée à être utilisée ou utilisation suivant l'une quelconque des revendications précédentes, dans laquelle la maladie ou le trouble est une maladie auto-immune ou un trouble auto-immun.

20. Composition destinée à être utilisée ou utilisation suivant la revendication 19, dans laquelle la maladie auto-immune est la polyarthrite rhumatoïde.

21. Composition destinée à être utilisée ou utilisation suivant la revendication 19, dans laquelle la maladie auto-immune est le lupus érythémateux disséminé.

22. Composition destinée à être utilisée ou utilisation suivant l'une quelconque des revendications précédentes, dans laquelle l'anticorps dirigé contre CD40 est un anticorps monoclonal.

23. Composition destinée à être utilisée ou utilisation suivant la revendication 22, dans laquelle l'anticorps monoclonal dirigé contre CD40 est un anticorps chimère.

24. Composition destinée à être utilisée ou utilisation suivant la revendication 23, dans laquelle l'anticorps dirigé contre CD40 est un anticorps humanisé.

25. Composition destinée à être utilisée ou utilisation suivant la revendication 22, dans laquelle l'anticorps dirigé contre CD40 entre en compétition pour la liaison de CD40 avec l'anticorps monoclonal S2C6.

26. Composition destinée à être utilisée ou utilisation suivant la revendication 22, dans laquelle l'anticorps dirigé contre CD40 a les caractéristiques de liaison de l'anticorps monoclonal S2C6.

27. Composition destinée à être utilisée ou utilisation suivant l'une quelconque des revendications précédentes, dans laquelle l'anticorps dirigé contre CD20 est un anticorps monoclonal.

28. Composition destinée à être utilisée ou utilisation suivant la revendication 27, dans laquelle l'anticorps monoclonal dirigé contre CD20 est un anticorps chimère.

29. Composition destinée à être utilisée ou utilisation suivant la revendication 28, dans laquelle l'anticorps monoclonal dirigé contre CD20 est C2B8.
